# EUROPEAN PATENT SPECIFICATION

(11) **EP 1 374 788 B1**
(45) Date of publication and mention of the grant of the patent: **12.10.2011**
(21) Application number: 03253587.4
(22) Date of filing: 06.06.2003
(51) Int. Cl.: A61B 18/12

(54) **Electrosurgical system**
Elektrochirurgisches System
Système d'électrochirurgie

(30) Priority: 27.06.2002 GB 0214907
(43) Date of publication of application: 02.01.2004
(73) Proprietor: GYRUS MEDICAL LIMITED, Cardiff CF3 0LT (GB)
(72) Inventor: Fleming, Alistair Ian, Canton, Cardiff CF11 9NS (GB); Jones, Huw Leonard, Mid Glamorgan CF72 8PZ (GB)
(74) Representative: Blatchford, William Michael

(56) References cited:
- US-B1- 6 296 636

## Description

This invention as set out in the appended claims relates to an electrosurgical system, and in particular to one in which an electrosurgical generator provides a radio frequency (RF) cutting signal to a bipolar surgical instrument.

A typical bipolar cutting instrument, which may also be capable of tissue coagulation, comprises first and second electrodes separated by an insulating spacer. An early example of a bipolar RF cutting device is US 4,706,667 issued to Roos, in which the return or "neutral" electrode is set back from the active electrode. In a series of patents (including US 4,674,498, US 4,850,353, US 4,862,890 and US 4,958,539) Stasz proposed a variety of cutting blade designs. These were designed with relatively small gaps between two electrodes such that arcing would occur therebetween when an RF signal was applied to the blade, the arcing causing the cutting of the tissue. In an alternative arrangement, described in our co-pending patent applications GB 0130975.6 and US 10/105,811, a device is provided in which the spacing of the electrodes is designed such that direct arcing between the electrodes does not occur, but arcing does occur between one of the electrodes and the tissue at the target site.

Normal use of this instrument has proved very satisfactory, but in exceptional circumstances (especially where the instrument has been used in an overly aggressive manner) a situation hereinafter referred to as a "flare-out" may develop. It is not uncommon for small particles of condensed tissue and other debris to become attached to the electrodes, and ordinarily this poses no particular problem. However, in the case of a flare-out, the debris forms a conductive track between the electrodes, allowing current to flow directly therebetween. This low impedance electrical pathway from one electrode to the other, if allowed to continue for a period of several seconds, may conduct sufficient current that a failure of the device may occur. This may be by way of a failure of the insulating material forming the spacer, either by the insulating material experiencing such high temperatures that it becomes conductive, or by the temperature differentials throughout the insulator causing a physical cracking of the material. Alternatively, the extreme temperatures caused by the current flow may produce a physical melting of the electrode material itself.

In U.S. 6,296,636 (Cheng et al), there is disclosed an electrosurgical system in which high frequency energy from a generator is applied to an instrument having an array of electrodes each insulated from each other. Each electrode is fed from a power source isolated from the other electrodes, circuitry associated with the power source limiting or interrupting current flow to the electrode when low resistivity material (e.g. blood, saline irrigant, or conductive gel) causes, a low impedance path to exist between the respective electrode and an return electrode. In one variant, the generator is placed in a standby mode when the current output reaches 5 amps.

The present invention provides a way in which this condition, although rarely occurring, can be prevented from causing a failure of the device. Accordingly, there is provided an electrosurgical system including an RF generator; an electrosurgical instrument comprising at least first and second electrodes and an insulating spacer separating the first and second electrodes, the RF generator being adapted to supply a RF signal between the first and second electrodes, the signal being capable of causing tissue vaporisation means for measuring a characteristic of the output of the RF generator; and a controller adapted to analyse the measured characteristic and change the RF signal supplied between the first and second electrodes when an aspect of the characteristic exhibits rapid changes indicating the onset of a "flare-out" caused by debris forming a conductive track between the electrodes, allowing current to flow directly therebetween.

Conveniently, the characteristic of the output of the RF generator which is measured is the voltage across the first and second electrodes, or alternatively the current flowing therebetween. It has been discovered that there are a number of criteria which may indicate the onset of a flare-out. These include rapid changes in the impedance experienced between the electrodes, leading to large and sudden changes in the voltage between the electrodes or the current flowing therebetween. There may be an increase in the number or amplitude of high frequency components of the current or voltage signal, or an increase in the D.C. thermionic current sensed between the electrodes. In a preferred arrangement, the controller is responsive to the changeability of the measured characteristic as indicative of the onset of flare-out, typically the rate of change of the impedance between the electrodes, or the changeability as represented by the sum of the differences between successive impedance measurements.

Preferably, the controller is adapted to change the RF signal by reducing the power thereof when the aspect of the measured characteristic exhibits rapid changes. Alternatively, the controller may reduce the voltage of the radio frequency signal, or even the frequency thereof. Where the RF signal comprises a signal having dual components at a first and second frequency, the controller may change the signal by adjusting the relative proportions of the first and second frequency components. Preferably, however, the controller is adapted to reduce the power of the RF signal, and may reduce it substantially to zero in response to rapid changes of the measured characteristic. Conveniently, the power is reduced substantially to zero for a period of at least 5 seconds, allowing time for the instrument to be withdrawn from the surgical site and the electrodes to be cleaned if necessary. Alternatively the power is reduced to zero until the operator of the instrument manually resets the instrument.

In one convenient arrangement, the controller is adapted to reduce the power of the RF signal supplied between the first and second electrodes only when the aspect of the characteristic exhibits rapid changes for a predetermined period of time. This serves to ensure that false detection of a flare-out is not triggered by a transient change in the measured characteristic. The system may require a series of repeated measurements of the characteristic to all fit a predetermined criterion before action is taken.

Although potentially of use with other types of instrument, the present invention is primarily designed to be employed with instruments in which the first and second electrodes and the insulating spacer are such that the spacing between the electrodes is between 0.25 mm and 3.0 mm.

According to one preferred construction which does not form part of the invention, an electrosurgical system includes an RF generator, an electrosurgical instrument comprising at least first and second electrodes and an insulating spacer separating the first and second electrodes, the RF generator being adapted to supply a RF signal between the first and second electrodes, means for measuring the impedance between the first and second electrodes, and a controller adapted to analyse the impedance measurements and interrupt the radio frequency signal supplied between the first and second electrodes when the changeability of the impedance exceeds a predetermined threshold value.

Additionally described hereinafter is an electrosurgical system which does not form part of the invention and which comprises an RF generator having a pair of output terminals, and an electrosurgical instrument having a bipolar electrode assembly in the form of first and second electrodes adjacent each other and insulated from each other by a spacer, the electrodes being connectible to the output terminals, wherein the generator includes means for generating a monitoring signal representative of an electrical parameter associated with signals developed across the electrodes, and a controller responsive to the monitoring means to change the RF signal supplied by the generator to the instrument when the monitoring signal meets a predetermined criterion indicative of the onset of flare-out. Typically, the predetermined criterion is the variability of the electrical parameter or the monitoring signal reaching or exceeding a predetermined level. In the preferred generator, the electrical parameter is the load impedance across the generator output terminals and the controller is arranged to generate the sum of the differences between successive samples of the monitoring signal taken over a predetermined measurement period as a representation of the variability of the parameter.

The present invention is set out in the claims and will now be further described, by way of example only, with reference to the accompanying drawings in which,
Figure 1 is a schematic diagram of an electrosurgical system in accordance with the present invention;
Figure 2 is a schematic side view of an electrosurgical instrument suitable for use in the system of Figure 1,
Figure 3 is a schematic block diagram of the generator of the system of Figure 1, and
Figures 4 and 5 are schematic representations of the voltage, current and impedance measured across the electrodes of the system of Figure 1, in normal operation and in the event of a flare-out, respectively.

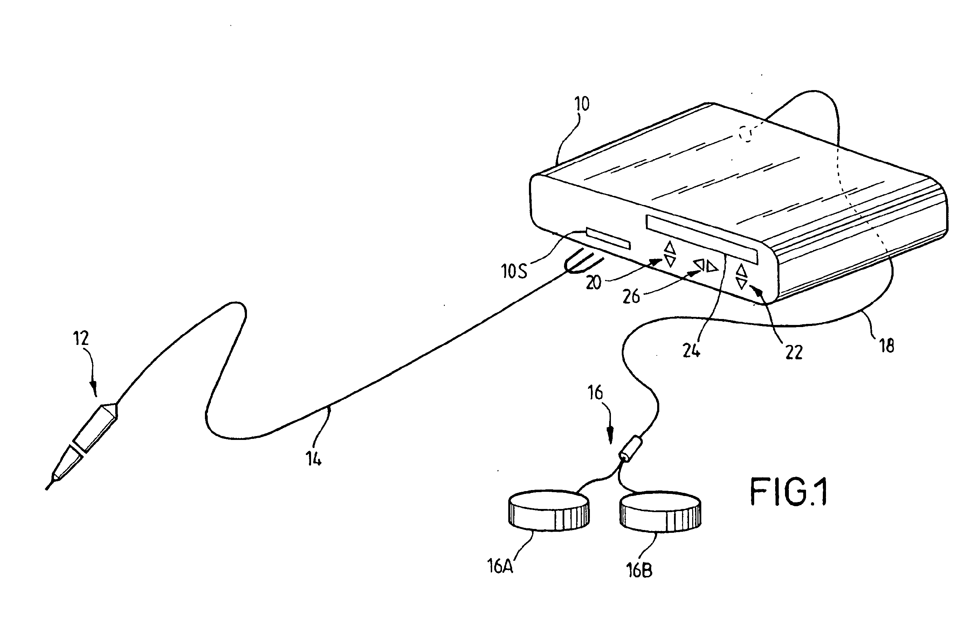

Referring to Figure 1, a generator 10 has an output socket 10S providing an RF output for an instrument 12 via a connection cord 14. Activation of the generator may be performed from the instrument 12 via a connection in cord 14 or by means of a footswitch unit 16, as shown, connected to the rear of the generator by a footswitch connection cord 18. Footswitch unit 16 has two footswitches 16A and 16B for selecting a coagulation mode and a cutting mode of the generator respectively. The generator front panel has push buttons 20 and 22 for respectively setting coagulation and cutting power levels, which are indicated in a display 24. Push buttons 26 are provided as an alternative means for selection between coagulation and cutting modes.

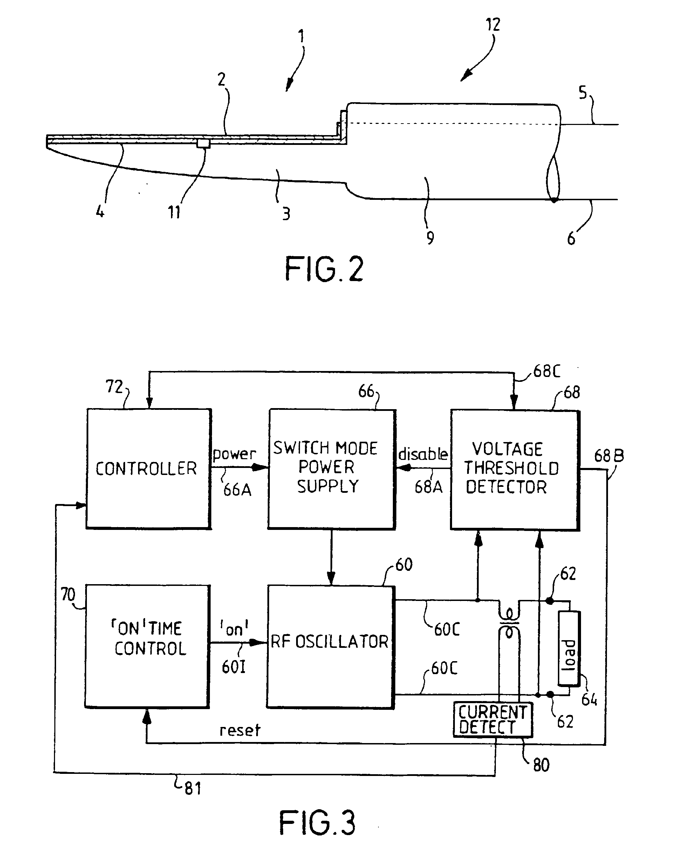

Referring to Figure 2, the instrument 12 comprises a blade shown generally at 1 and including a generally flat first electrode 2, a larger second electrode 3 and an electrical insulator 4 separating the first and second electrodes. The first electrode 2 is formed of stainless steel while the second electrode 3 is formed from copper integrally with a body portion 9. The surface of the second electrode is plated with a biocompatible material such as stainless steel, or alternatively with a non-oxidising material such as gold, platinum or palladium. The electrical insulator 4 is formed from a ceramic material such as Al₂O₃. A conductive lead 5 is connected to the first electrode 2, while another conductive lead 6 is connected to the second electrode 3. The RF output from the generator 10 is connected to the blade 1 via the leads 5 and 6 so that a radio frequency signal having a substantially constant peak voltage (typically around 400V) appears between the first and second electrodes. When the blade 1 is brought into contact with tissue at a target site, the RF voltage causes arcing between one of the electrodes and the tissue surface. Because the first electrode 2 is smaller in cross-sectional area, and has a lower thermal capacity and conductivity than that of the second electrode 3, the first electrode assumes the role of the active electrode and arcing occurs from this electrode to the tissue. Electrical current flows through the tissue to the second electrode 3, which assumes the role of the return electrode. Cutting of the tissue occurs at the active electrode, and the blade may be moved through the tissue.

Referring to Figure 3, the generator comprises an RF power oscillator 60 having a pair of output lines 60C for coupling via output terminals 62 to the load impedance 64 represented by the instrument 12 when in use. Power is supplied to the oscillator 60 by a switched mode power supply 66.

In an example, the RF oscillator 60 operates at about 400kHz, with any frequency from 300kHz upwards into the HF range being feasible. The switched mode power supply typically operates at a frequency in the range of from 25 to 50kHz. Coupled across the output lines 60C is a voltage threshold detector 68 having a first output 68A coupled to the switched mode power supply 16 and a second output 68B coupled to an "on" time control circuit 70. A microprocessor controller 72 coupled to the operator controls and display (shown in Figure 1) is connected to a control input 66A of the power supply 66 for adjusting the generator output power by supply voltage variation and to a threshold-set input 68C of the voltage threshold detector 68 for setting peak RF output voltage limits. Also coupled across the output lines 60C is a current detection circuit 80 which feeds signals to the controller 72 via line 81.

In operation, the microprocessor controller 72 causes power to be applied to the switched mode power supply 66 when electrosurgical power is demanded by the surgeon operating an activation switch arrangement which may be provided on a handpiece or footswitch (see Figure 1). A constant output voltage threshold is set independently on the supply voltage via input 68C according to control settings on the front panel of the generator (see Figure 1). Typically, for desiccation or coagulation the threshold is set at a desiccation threshold value between 150 volts and 200 volts. When a cutting or vaporisation output is required the threshold is set to a value in the range of from 250 or 300 volts to 600 volts. These voltage values are peak values. Their being peak values means that for desiccation at least it is preferable to have an output RF waveform of low crest factor to give maximum power before the voltage is clamped at the values given. Typically a crest factor of 1.5 or less is achieved.

When the generator is first activated, the status of the control input 60I of the RF oscillator 60 (which is connected to the "on" time control circuit 70) is "on", such that the power switching device which forms the oscillating element of the oscillator 60 is switched on for a maximum conduction period during each oscillation cycle. The power delivered to the load 64 depends partly on the supply voltage applied to the RF oscillator 60 from the switched mode power supply 66 and partly on the load impedance 64. The voltage threshold for a desiccation output is set to cause trigger signals to be sent to the "on" time control circuit 70 and to the switched mode power supply 66 when the voltage threshold is reached. The "on" time control circuit 70 has the effect of virtually instantaneously reducing the "on" time of the RF oscillator-switching device. Simultaneously, the switched mode power supply is disabled so that the voltage supplied to oscillator 60 begins to fall. The operation of the generator in this way is described in detail in our European Patent Application No. 0754437.

Referring back to Figure 2, when the instrument 12 is in use, small particles of condensed tissue and other debris can become adhered to the edge electrode 2 and, to a lesser extent, the base electrode 3. If the instrument is used particularly aggressively, it is possible that a conductive track of such debris can build up between the electrodes 2 and 3 across the ceramic insulator 4. Such a conductive track is shown schematically at 11 in Figure 2. If no action is taken to prevent it, this conductive track 11 develops into a "flare-out" in which the current passing directly between the electrode 2 and the electrode 3 will cause the instrument to overheat and finally fail. The following description explains how the generator 10 detects and compensates for just such a situation.

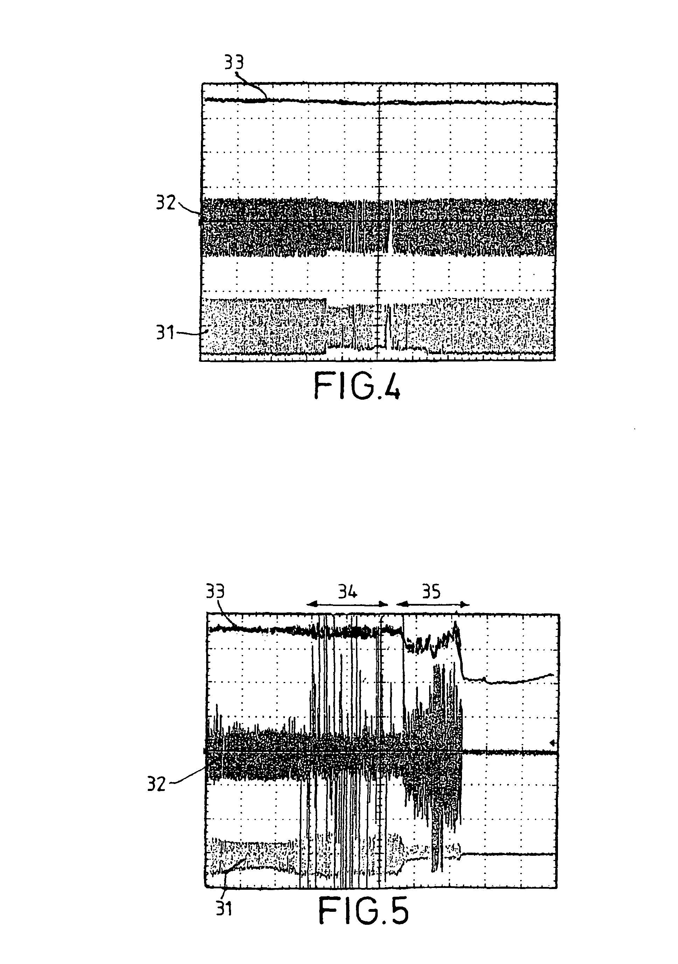

At regular intervals, in this case every 10ms, the current is measured across the load 64 by the current detector 80 and the current value is sent to the controller 72. The controller uses the current value to determine repeatedly the impedance across the load 64. The difference between successive impedance values is calculated, and summed for 16 consecutive readings to give a first total Z₁ The current measurements continue every 10ms until a further 16 consecutive impedance calculations have been made, which calculations are again summed to give a second total Z₂. If Z₁ and Z₂ are both less than the threshold criteria for the sum Q of the impedance changes, then the generator continues to supply RF signals to the instrument 12. The process is continued with further current measurements being sent to the controller 72 every 10ms. This normal operation is shown in Figure 4, in which the voltage across the electrodes 2, 3 is shown by trace 31, the current flowing by trace 32 and the impedance measured by the generator by trace 33.

If a flare-out starts to develop between the electrodes 2 and 3, the current measured across the load 64 starts to fluctuate widely, and with a high frequency of oscillation. This is shown in Figure 5, with the build up to the flare-out being shown at 34 and the onset of the flare-out at 35. In these circumstances Z₁ and Z₂ are both above the threshold for the sum Q of the impedance changes, and this causes the controller to send a signal to the power supply 66 to cause the power to be interrupted. A typical value for Q is 1000 ohms, for a 16 measurement cycle.

In addition to interrupting the power supply, the controller may cause a message (such as "Clean Tip") to be displayed by the display 24. The controller does not allow power to be restored to the output of the generator until the surgeon has pressed a reset button to indicate that the tip has been cleaned, and will repeat the interruption process if the impedance measurements show that the flare-out conditions are still in existence when the power is recommenced.

It will be appreciated that criteria other than the changeability of the impedance across the output of the generator could be employed to give an indication of the onset of a flare-out. These include, non-exhaustively, the high frequency content (e.g. the number of high frequency components) of the modulation of the current or voltage signal, or the D.C. thermionic current flowing between the electrodes 2 and 3. The latter can be measured in the manner disclosed in European Patent Publication No. 1053719.

Some aspects of the present description could also be used to prevent overheating of electrodes without the actual existence of a flare-out. The generator, detecting that a criterion indicating the start of a potential overheating situation has been met, could reduce the power or alter the radio frequency signal in other ways so as to maintain operation of the electrosurgical system within proper parameters. Those skilled in the art of electrosurgical generators wil readily be able to establish suitable detection criteria to keep the generator operating within safe and effective limits. However, the present invention is set out in the appended claims.

## Claims

1. An electrosurgical system including:
a radio frequency generator (10);
an electrosurgical instrument (12) comprising at least first and second electrodes (2, 3) and an insulating spacer (4) separating the first and second electrodes, the radio frequency generator being adapted to supply a radio frequency signal between the first and second electrodes, the signal being capable of causing tissue vaporisation;
means for measuring a characteristic of the output of the radio frequency generator;
a controller (72) adapted to analyse the measured characteristic and change the radio frequency signal supplied between the first and second electrodes **characterised in that** the controller changes said radio frequency signal when an aspect of the characteristic exhibits rapid changes indicating the onset of a "flare-out" caused by debris forming a conductive track between the electrodes (2,3), allowing current to flow directly therebetween.

2. An electrosurgical system according to claim 1, wherein the characteristic of the output of the radio frequency generator (10) is the voltage across the first and second electrodes.

3. An electrosurgical system according to claim 1, wherein the characteristic of the output of the radio frequency generator (10) is the current flowing between the first and second electrodes.

4. An electrosurgical system according to any preceding claim,
wherein the controller (72) is responsive to the changeability of the measured characteristic to change the said radio frequency signal.

5. An electrosurgical system according to claim 4, wherein the changeability of the measured characteristic is represented by the sum of the differences between successive measurements of the characteristic.

6. An electrosurgical system according to any preceding claim, wherein the controller (72) is adapted to change the radio frequency signal by reducing the power of the radio frequency signal when the aspect of the characteristic exhibits rapid changes.

7. An electrosurgical system according to any preceding claim, wherein the controller (72) is adapted to reduce the power of the radio frequency signal substantially to zero when the aspect of the characteristic exhibits rapid changes.

8. An electrosurgical system according to claim 7, wherein the controller (72) is adapted to reduce the power of the radio frequency signal substantially to zero for a minimum period of 5 seconds.

9. An electrosurgical system according to claim 7, wherein the controller (72) is adapted to reduce the power of the radio frequency signal substantially to zero until a user of the system undertakes a manual reset operation.

10. An electrosurgical system according to any preceding claim, wherein the controller (72) is adapted to reduce the power of the radio frequency signal supplied between the first and second electrodes only when the aspect of the characteristic exhibits rapid changes for a predetermined period of time.

11. An electrosurgical system according to any preceding claim, wherein the first and second electrodes (2, 3) and the insulating spacer (4) are such that the spacing between the first and second electrodes is between 0.25mm and 3.0mm.

## Patentansprüche

1. Elektrochirurgisches System, umfassend:
einen Funkfrequenzgenerator (10);
ein elektrochirurgisches Instrument (12) mit mindestens einer ersten und
einer zweiten Elektrode (2, 3) und mit einem isolierenden Distanzhalter (4), der die erste Elektrode und die zweite Elektrode voneinander trennt, wobei der Funkfrequenzgenerator ausgebildet ist für die Bereitstellung eines Funkfrequenzsignals zwischen der ersten und der zweiten Elektrode, wobei das Signal geeignet ist, eine Verdampfung von Gewebe zu bewirken;
Mittel zum Messen einer Charakteristik der Ausgabe des Funkfrequenzgenerators; und
einen Controller (72), der ausgebildet ist für die Analyse der gemessenen Charakteristik und für die Änderung des zwischen der ersten und der zweiten Elektrode bereitgestellen Funkfrequenzsignals, **dadurch gekennzeichnet, dass** der Controller das Funkfrequenzsignal ändert, wenn ein Aspekt der Charakteristik rasche Veränderungen zeigt, die auf den Beginn einer durch Ablagerungen verursachten Streuung hinweisen, die eine leitende Bahn zwischen den Elektroden (2, 3) bildet, so dass direkt zwischen den Elektroden elektrischer Strom fließen kann.

2. Elektrochirurgisches System nach Anspruch 1, wobei die Charakteristik der Ausgabe des Funkfrequenzgenerators (10) eine Spannung über der ersten und der zweiten Elektrode ist.

3. Elektrochirurgisches System nach Anspruch 1, wobei die Charakteristik der Ausgabe des Funkfrequenzgenerators (10) der zwischen der ersten rund der zweiten Elektrode fließende Strom ist.

4. Eiektrochirurgisches System nach einem der vorhergehenden Ansprüche, wobei der Controller (72) auf eine Veranderlichkeit der gemessenen Charakteristik reagiert, um das Funkfrequenzsignal zu ändern.

5. Elektrochirurgisches System nach Anspruch 4, wobei die Veranderlichkeit der gemessenen Charakteristik durch die Summe der Differenz zwischen aufeinanderfolgenden Messungen der Charakteristik dargestellt wird.

6. Elektrochirurgisches System nach einem der vorhergehenden Ansprüche, wobei der Controller (72) ausgebildet ist für die Änderung des Funkfrequenzsignals durch eine Reduzierung der Leistung des Funkfrequenzsignals, wenn der Aspekt der Charakteristik rapide Veränderungen zeigt.

7. Elektrochirurgisches System nach einem der vorhergehenden Ansprüche, wobei der Controller (72) ausgebildet ist für die Reduzierung der Leistung des Funkfrequenzsignals auf im Wesentlichen Null wenn der Aspekt der Charakteristik rapide Veränderungen zeigt.

8. Elektrochirurgisches System nach Anspruch 7, wobei der Controller (72) ausgebildet ist, die Leitung des Funkfrequenzsignals für eine minimale Dauer von fünf Sekunden auf im Wesentlichen Null zu reduzieren.

9. Eiektrochirurgisches System nach Anspruch 7, wobei der Controller (72) ausgebildet ist für die Reduzierung der Leistung des Funkfrequenzsignals auf im Wesentlichen Null, bis ein Benutzer des Systems eine manuelle Rückstellung des Systems durchführt.

10. Elektrochirurgisches System nach einem der vorhergehenden Ansprüche, wobei der Controller (72) ausgebildet ist für die Reduzierung der Leitung des zwischen der ersten und der zweien Elektrode bereitgestellten Funkfrequenzsignals nur dann, wenn der Aspekt der Chrakteristik für eine vorgegebene Zeitdauer rapide Veränderungen zeigt.

11. Elektrochirurgiches System nach einem der vorhergehenden Anspruche, wobei die erste und die zweite Elektrode (2, 3) und der isolierende Distanzhalter (4) dergestalt sind, dass der Abstand zwischen der ersten und der zweiten Elektrode zwischen 0,25 mm und 3,0 mm beträgt.

## Revendications

1. Système d'électrochirurgie comprenant :
un générateur à haute fréquence (10) ;
un instrument électrochirurgical (12) comprenant au moins des première et deuxième électrodes (2, 3) et un dispositif d'espacement isolant (4) séparant les première et deuxième électrodes, le générateur à haute fréquence étant adapté pour fournir un signal de haute fréquence entre les première et deuxième électrodes, le signal pouvant provoquer une vaporisation du tissu ;
des moyens pour mesurer une caractéristique de la sortie du générateur à haute fréquence ;
un contrôleur (72) adapté pour analyser la caractéristique mesurée et changer le signal de haute fréquence fourni entre les première et deuxième électrodes, **caractérisé en ce que** le contrôleur change ledit signal de haute fréquence, lorsqu'un aspect de la caractéristique laisse apparaître des changements rapides indiquant le début d'un « arrondi » provoqué par les débris formant une voie conductrice entre les électrodes (2, 3), permettant au courant de s'écouler directement entre elles.

2. Système d'électrochirurgie selon la revendication 1, dans lequel la caractéristique de la sortie du générateur à haute fréquence (10) est la tension sur les première et deuxième électrodes.

3. Système d'électrochirurgie selon la revendication 1, dans lequel la caractéristique de la sortie du générateur à haute fréquence (10) est le courant s'écoulant entre les première et deuxième électrodes.

4. Système d'électrochirurgie selon l'une quelconque des revendications précédentes, dans lequel le contrôleur (72) est sensible au changement de la caractéristique mesurée pour changer ledit signal de haute fréquence.

5. Système d'électrochirurgie selon la revendication 4, dans lequel le changement de la caractéristique mesurée est répété par la somme de différences entre les mesures successives de la caractéristique.

6. Système d'électrochirurgie selon l'une quelconque des revendications précédentes, dans lequel le contrôleur (72) est adapté pour changer le signal de haute fréquence en réduisant la puissance du signal de haute fréquence lorsque l'aspect de la caractéristique laisse apparaître des changements rapides.

7. Système d'électrochirurgie selon l'une quelconque des revendications précédentes, dans lequel le contrôleur (72) est adapté pour réduire la puissance du signal de haute fréquence sensiblement à zéro lorsque l'aspect de la caractéristique laisse apparaître des changements rapides.

8. Système d'électrochirurgie selon la revendication 7, dans lequel le contrôleur (72) est adapté pour réduire la puissance du signal de haute fréquence sensiblement à zéro pendant une période minimum de 5 secondes.

9. Système d'électrochirurgie selon la revendication 7, dans lequel le contrôleur (72) est adapté pour réduire la puissance du signal de haute fréquence sensiblement à zéro jusqu'à ce qu'un utilisateur du système se charge d'une opération de réinitialisation manuelle.

10. Système d'électrochirurgie selon l'une quelconque des revendications précédentes, dans lequel le contrôleur (72) est adapté pour réduire la puissance du signal de haute fréquence fourni entre les première et deuxième électrodes, uniquement lorsque l'aspect de la caractéristique laisse apparaître des changements rapides pendant une période de temps prédéterminée.

11. Système d'électrochirurgie selon l'une quelconque des revendications précédentes, lorsque les première et deuxième électrodes (2, 3) et le dispositif d'espacement isolant (4) sont tels que l'espacement entre les première et deuxième électrodes est compris entre 0,25 mm et 3,0 mm.
